# EUROPÄISCHE PATENTSCHRIFT

(11) **EP 2 389 861 B1**
(45) Veröffentlichungstag und Bekanntmachung des Hinweises auf die Patenterteilung: **24.07.2013**
(21) Anmeldenummer: 11400024.3
(22) Anmeldetag: 24.03.2011
(51) Int. Cl.: A61B 5/107, G01B 5/06

(54) **Vorrichtung zur Längenmessung**
Length measuring device
Dispositif de mesure des longueurs

(30) Priorität: 31.05.2010 DE 102010022638
(43) Veröffentlichungstag der Anmeldung: 30.11.2011
(73) Patentinhaber: seca ag, 4153 Reinach BL 1 (CH)
(72) Erfinder: Winterberg, Matthias, 21029 Hamburg (DE); Torkler, Michael, 23847 Gross Boden (DE); Schielmann, Sven, 23795 Bad Segeberg (DE)
(74) Vertreter: Klickow, Hans-Henning

(56) Entgegenhaltungen:
- DE-A1- 19 808 432
- DE-U- 1 984 779
- DE-U1- 29 713 667
- US-A1- 2005 155 246

## Beschreibung

Die Erfindung betrifft eine Vorrichtung zur Längenmessung, die einen Längenmessstab mit Halteelementen für eine Wandmontage mit einer Messeinrichtung sowie ein Basiselement mit einer Standfläche für eine zu vermessende Person aufweist, sowie bei der das Basiselement relativ zum Längenmessstab fixierbar ist.

Bei der Anwendung derartiger Vorrichtungen ist es zur Erreichung ausreichend exakter Messergebnisse erforderlich, daß die zu vermessende Person eine korrekte Lage relativ zum Längenmessstab einnimmt. Im Bereich der Standfläche können deshalb Positionierhilfen angeordnet werden. Die Positionierhilfen können beispielsweise Markierungen im Bereich der Standfläche und/oder ein Fersenanschlag sein.

Bei einem Transport der Vorrichtung werden typischerweise der Längenmessstab und das Basiselement voneinander getrennt, um eine kompakte Transportdimensionierung zu unterstützen. Bei einer Inbetriebnahme ist es deshalb erforderlich, das Basiselement mit dem Längenmessstab zu verbinden. Die Verbindung soll dabei derart erfolgen, daß das Basiselement ausreichend fixiert ist, um auch bei einem Betreten durch eine Person und/oder bei seitlichen Stoßbelastungen eine exakte Positionierung beizubehalten.

Aus der DE 197 13 667 U1 ist eine Vorrichtung zur Längenmessung bekannt, die einen Längenmessstab mit einer Messeinrichtung sowie ein Basiselement mit einer Standfläche aufweist. Das Basiselement ist relativ zum Längenmessstab fixierbar.

Eine ähnliche Vorrichtung wird auch in der US 2005/155246 A1 beschrieben.

Aus der DE 19 84 779 U ist eine weitere Vorrichtung zur Längenmessung mit einer Messeinrichtung bekannt. Ein Basiselement ist relativ zum Längenmessstab fixierbar.

Auch die DE 198 08 432 A1 erläutert eine Vorrichtung zur Längenmessung mit einem Längenmessstab und einer Messeinrichtung.

Aufgabe der vorliegenden Erfindung ist es daher, eine Vorrichtung der einleitend genannten Art derart zu konstruieren, daß eine einfache Montage und Demontage des Basiselementes unterstützt wird und eine zuverlässige Positionsfestlegung des Basiselementes erreicht wird.

Diese Aufgabe wird erfindungsgemäß dadurch gelöst, daß das Basiselement durch mindestens ein am Längenmessstab beweglich geführtes Andruckelement zwischen Andruckelement und Fußboden festklemmbar ist.

Durch die Festklemmbarkeit des Basiselementes ist es in einfacher weise und mit einem geringen Zeitaufwand möglich, eine Verbindung zwischen dem Basiselement und dem Längenmessstab herzustellen. Darüber hinaus wird das Basiselement relativ zur Aufstellfläche sichtbar fixiert, so daß die Positionierung des Basiselementes auch bei einer Benutzung sicher beibehalten wird. Durch die Möglichkeit einer schnellen Montage und Demontage des Basiselementes wird eine Reinigung eines Fußbodens unter dem Basiselement unterstützt. Vorzugsweise wird das Basiselement zwischen dem Andruckelement und dem Fußboden festgeklemmt.

Hohe Andruckkräfte können dadurch bereitgestellt werden, daß das Andruckelement von mindestens einer Feder gegen das Basiselement gedrückt ist.

Gemäß einer vereinfachten Ausführungsvariante ist auch daran gedacht, daß das Andruckelement durch die Einwirkung der Schwerkraft gegen das Basiselement gedrückt ist.

Eine zuverlässige Positionsfestlegung des Basiselementes kann dadurch erfolgen, daß das Basiselement formschlüssig mit dem Andruckelement verbunden ist.

Eine einfache Montage wird dadurch unterstützt, daß das Basiselement in einem teilmontiertem Zustand relativ zum Andruckelement verschwenkbar angeordnet ist.

Eine definierte Positionierung der zu vermessenden Person wird dadurch erleichtert, daß das Andruckelement einen Fersenanschlag bereitstellt.

Eine stabile Ausführungsform wird dadurch bereitgestellt, daß das Andruckelement mindestens bereichsweise als ein L-Profil ausgebildet ist. Ein anderes Ausführungsbeispiel betrifft eine T-Form.

Die bewegliche Führung des Andruckelementes kann dadurch realisiert werden, daß das Andruckelement von mindestens einem Ausrichtelement im Bereich des Längenmessstabes geführt ist.

Eine kompakte Ausführungsform wird dadurch bereitgestellt, daß im Bereich des Ausrichtelementes mindestens eine Feder angeordnet ist.

Unbeabsichtigte Drehbewegungen können dadurch vermieden werden, daß das Andruckelement von mindestens zwei Ausrichtelementen im Bereich des Längenmessstabes geführt ist. Grundsätzlich ist aber ein Ausrichtelement ausreichend.

Eine kompakte und zugleich stabile Ausführungsform wird dadurch bereitgestellt, daß das Ausrichtelement in einem mindestens bereichsweise von einer Wandung des Längenmessstabes umschlossenen Aufnahmeraum angeordnet ist, der beispielsweise durch ein Hohlprofil bereitgestellt wird. Gemäß einer anderen Ausführungsvariante ist auch daran gedacht, daß das Ausrichtelement außerhalb einer Wandung des Längenmessstabes angeordnet ist.

In den Zeichnungen sind Ausführungsbeispiele der Erfindung schematisch dargestellt. Es zeigen:
- Fig. 1: eine perspektivische Darstellung eines Längenmessstabes mit Kopfanschlag sowie einem Basiselement mit Standfläche,
- Fig. 2: die Vorrichtung gemäß Fig. 1 mit vom Längenmesstab getrenntem Basiselement,
- Fig. 3: die Vorrichtung gemäß Fig. 2 nach einer Teilmontage des Basiselementes und
- Fig. 4: eine teilweise Darstellung eines Längsschnittes durch den Längenmessstab im Bereich eines Andruckelementes für das Basiselement.

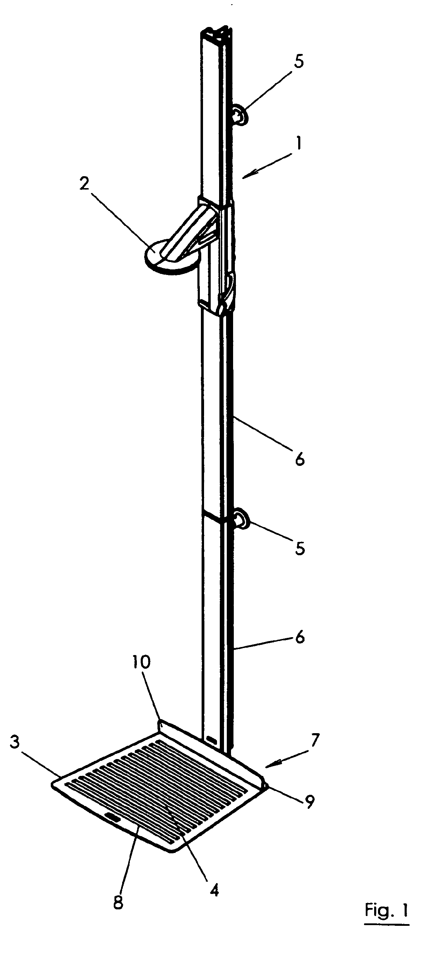

Gemäß der Ausführungsform in Fig. 1 ist ein Längenmessstab (1) mit einem Kopfanschlag (2) für den Kopf einer zu vermessenden Person sowie einem Basiselement (3) versehen, das eine Standfläche (4) für die zu vermessende Person aufweist. Der Längenmessstab (1) weist Halteelemente (5) zur Unterstützung einer Wandmontage auf. Beim dargestellten Ausführungsbeispiel besteht der Längenmessstab (1) aus einzelnen Stabsegmenten (6), die modulartig miteinander kombiniert werden können.

Das Basiselement (3) wird unter Verwendung eines Andruckelementes (7) positioniert, das beweglich im Bereich des Längenmessstabes (1) geführt ist. Im Bereich der Standfläche (4) ist vorzugsweise eine Oberflächenprofilierung (8) angeordnet, um ein sicheres Stehen der zu vermessenden Person zu unterstützen. Die Oberlächenprofilierung kann beispielsweise in Form von Fußumrissen realisiert werden.

Beim dargestellten Ausführungsbeispiel ist das Andruckelement (7) winkelartig ausgebildet und weist einen Horizontalschenkel (9) sowie einen Vertikalschenkel (10) auf. Der Vertikalschenkel (10) dient als Fersenanschlag.

Das Basiselement (3) kann aus einem festen oder flexiblen Material ausgebildet sein. Gedacht ist insbesondere an die Verwendung von Kunststoffen oder elastomeren Materialien.

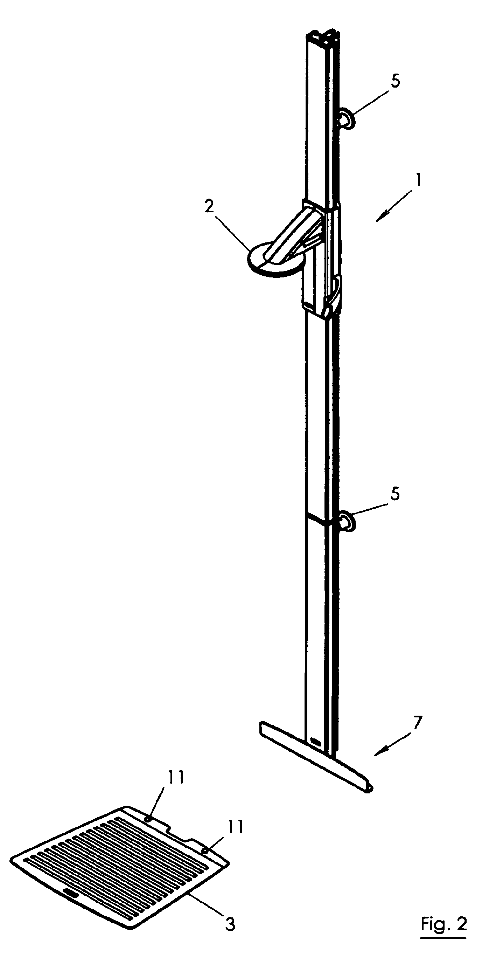

Fig. 2 zeigt das Basiselement (3) in einem vom Andruckelement (7) getrennten Zustand. Es ist zu erkennen, daß das Basiselement (3) beim dargestellten Ausführungsbeispiel zwei Verbindungsprofile (11) aufweist. Die Verbindungsprofile (11) sind angepaßt an Gegenprofile (12) ausgebildet, die im Bereich des Andruckelementes (7) angeordnet, allerdings in Fig. 2 nicht erkennbar sind. Das Verbindungsprofil (11) kann beispielsweise als eine Ausnehmung oder ein zapfen ausgebildet sein.

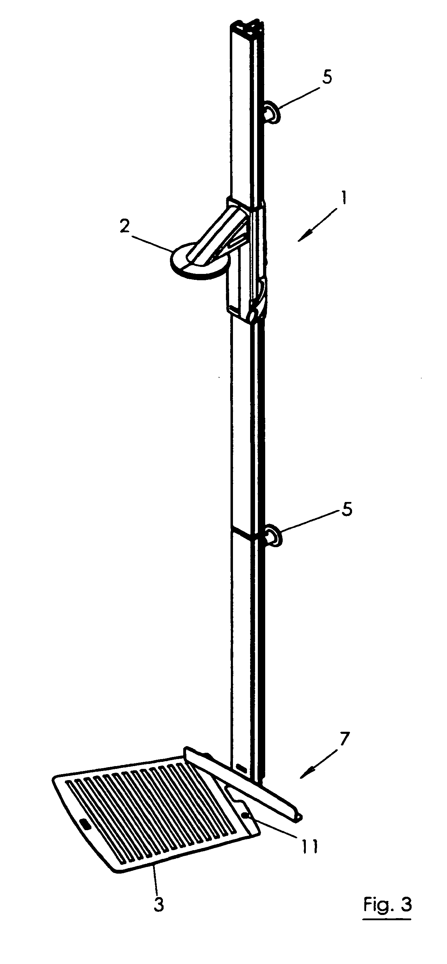

Fig. 3 zeigt einen teilmontierten Zustand des Basiselementes (3). Das Basiselement (3) ist hierbei bereits mit einem Verbindungsprofil (11) in das in Fig. 3 ebenfalls nicht erkennbare Gegenprofil (12) des Andruckelementes (7) eingefügt worden. Dieser Verbindungsvorgang erfolgt vorzugsweise in einer relativ zum Andruckelement (7) horizontal verschwenkten Positionierung des Basiselementes (3). Nach einem Verschwenken des Basiselementes (3) in den Grundzustand gemäß Fig. 1 sind dann weitere Verbindungsprofile (11) zu den jeweiligen Gegenprofilen (12) ausgerichtet.

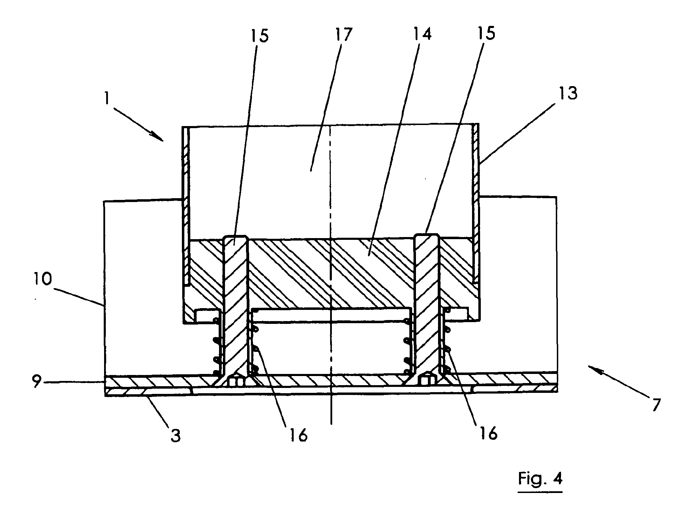

Fig. 4 zeigt in einem Längsschnitt die verschiebliche Führung des Andruckelementes (7) im Bereich des Längenmessstabes (1). Unterhalb des Andruckelementes (7) ist das Basiselement (3) angeordnet.

Aus Fig. 4 ist zu erkennen, daß der Längenmessstab (1) eine Wandung (13) aufweist. Die Wandung (13) haltert ein Lager (14), in dem das Andruckelement (7) mit mindestens einem Ausrichtelement (15) geführt ist. Beim dargestellten Ausführungsbeispiel werden zwei Ausrichtelemente (15) verwendet. Die Ausrichtelemente (15) können bolzenartig ausgebildet sein.

Gemäß dem Ausführungsbeispiel in Fig. 4 stützt sich der Horizontalschenkel (9) mit Federn (16) gegenüber dem Längenmessstab (1) ab. Bei einer Verschiebung des Andruckelementes (7) entlang einer Längsachse (17) des Längenmessstabes werden die Federn (16) zusammengedrückt und es ist möglich, das Basiselement (3) unterhalb des Andruckelementes (7) zu positionieren. Nach einer entsprechenden Positionierung des Basiselementes (3) drücken die Federn (16) das Andruckelement (7) gegen das Basiselement (3) und fixieren hierdurch das Basiselement (3) im Bereich einer Aufstellfläche.

## Patentansprüche

1. Vorrichtung zur Längenmessung, die einen Längenmessstab (1) mit Halteelementen (5) zur Wandmontage mit einer Messeinrichtung sowie ein Basiselement (3) mit einer Standfläche (4) für eine zu vermessende Person aufweist, sowie bei der das Basiselement (3) relativ zum Längenmessstab (1) fixierbar ist, **dadurch gekennzeichnet, daß** das Basiselement (3) durch mindestens ein am Längenmessstab (1) beweglich geführtes Andruckelement (7) zwischen Andruckelement (7) und Fußboden festklemmbar ist.

2. Vorrichtung nach Anspruch 1, **dadurch gekennzeichnet, daß** das Andruckelement (7) von mindestens einer Feder (16) gegen das Basiselement (3) gedrückt ist.

3. Vorrichtung nach Anspruch 1, **dadurch gekennzeichnet, daß** das Andruckelement durch die Einwirkung der Schwerkraft gegen das Basiselement (3) gedrückt ist.

4. Vorrichtung nach einem der Ansprüche 1 bis 3, **dadurch gekennzeichnet, daß** das Basiselement (3) formschlüssig mit dem Andruckelement (7) verbunden ist.

5. verfahren nach einem der Ansprüche 1 bis 4, **dadurch gekennzeichnet, daß** das Basiselement (3) in einem teilmontiertem Zustand relativ zum Andruckelement (7) verschwenkbar angeordnet ist.

6. Vorrichtung nach einem der Ansprüche 1 bis 5, **dadurch gekennzeichnet, daß** das Andruckelement (7) einen Fersenanschlag bereitstellt.

7. Vorrichtung nach einem der Ansprüche 1 bis 6, **dadurch gekennzeichnet, daß** das Andruckelement (7) mindestens bereichsweise als ein L-Profil ausgebildet ist.

8. Vorrichtung nach einem der Ansprüche 1 bis 7, **dadurch gekennzeichnet, daß** das Andruckelement (7) von mindestens einem Ausrichtelement (15) im Bereich des Längenmessstabes (1) geführt ist.

9. Vorrichtung nach Anspruch 8, **dadurch gekennzeichnet, daß** im Bereich des Ausrichtelementes (15) mindestens eine Feder (16) angeordnet ist.

10. Vorrichtung nach Anspruch 8, **dadurch gekennzeichnet, daß** das Andruckelement (7) von mindestens zwei Ausrichtelementen (15) im Bereich des Längenmessstabes (1) geführt ist.

11. Vorrichtung nach einem der Ansprüche 8 bis 10, **dadurch gekennzeichnet, daß** das Ausrichtelement (15) in einem mindestens bereichsweise von einer Wandung (13) des Längenmessstabes (1) umschlossenen Aufnahmeraum angeordnet ist.

12. Vorrichtung nach einem der Ansprüche 8 bis 10, **dadurch gekennzeichnet, daß** das Ausrichtelement (15) außerhalb einer Wandung (13) des Längenmessstabes (1) angeordnet ist.

13. Vorrichtung nach einem der Ansprüche 1 bis 12, **dadurch gekennzeichnet, daß** das Andruckelement (7) am Basiselement (3) befestigt und zur Beaufschlagung des Längenmeßstabes (1) ausgebildet ist.

## Claims

1. Device for measuring height, which has a height-measuring scale (1) with retaining elements (5) for wall mounting, with a measuring apparatus and also a base element (3) with a stand-on surface (4) for a person who is to be measured, and in which the base element (3) can be fixed, relative to the height-measuring scale (1), **characterised in that** the base element (3) can be clamped fast, by at least one pressure element (7) which is movably guided on the height-measuring scale (1), between said pressure element (7) and the floor.

2. Device according to claim 1, **characterised in that** the pressure element (7) is pressed against the base element (3) by at least one spring (16).

3. Device according to claim 1, **characterised in that** the pressure element is pressed against the base element (3) by the action of gravity.

4. Device according to one of claims 1 to 3, **characterised in that** the base element (3) is connected to the pressure element (7) in a form-locking manner.

5. Device according to one of claims 1 to 4, **characterised in that** the base element (3) is arranged so as to be swivellable, relative to the pressure element (7), when in a partially assembled state.

6. Device according to one of claims 1 to 5, **characterised in that** the pressure element (7) provides a heel stop.

7. Device according to one of claims 1 to 6, **characterised in that** the pressure element (7) is constructed, at least in certain regions, as an L-section.

8. Device according to one of claims 1 to 7, **characterised in that** the pressure element (7) is guided by at least one aligning element (15) in the region of the height-measuring scale (1).

9. Device according to claim 8, **characterised in that** at least one spring (16) is arranged in the region of the aligning element (15).

10. Device according to claim 8, **characterised in that** the pressure element (7) is guided by at least two aligning elements (15) in the region of the height-measuring scale (1).

11. Device according to one of claims 8 to 10, **characterised in that** the aligning element (15) is arranged in a receiving compartment which is enclosed, at least in certain regions, by a wall (13) of the height-measuring scale (1).

12. Device according to one of claims 8 to 10, **characterised in that** the aligning element (15) is arranged outside a wall (13) of the height-measuring scale (1).

13. Device according to one of claims 1 to 12, **characterised in that** the pressure element (7) is fastened to the base element (3) and is constructed for the purpose of acting upon the height-measuring scale (1).

## Revendications

1. Dispositif pour la mesure de longueurs, qui est doté d'une toise (1), avec des éléments de fixation (5) pour le montage mural, avec un dispositif de mesure, ainsi que d'un élément de base (3), avec un plateau (4) pour une personne à mesurer, et dans lequel l'élément de base (3) peut être fixé par rapport à la toise (1), **caractérisé en ce que** l'élément de base (3) peut être serré fixement, au moyen d'au moins un élément de pression (7), entre ledit élément de pression (7) et le sol.

2. Dispositif selon la revendication 1, **caractérisé en ce que** l'élément de pression (7) est pressé contre l'élément de base (3) par au moins un ressort (16).

3. Dispositif selon la revendication 1, **caractérisé en ce que** l'élément de pression est pressé contre l'élément de base (3) sous l'effet de la pesanteur.

4. Dispositif selon l'une des revendications 1 à 3, **caractérisé en ce que** l'élément de base (3) est relié par emboîtement à l'élément de pression (7).

5. Dispositif selon l'une des revendications 1 à 4, **caractérisé en ce que**, dans une position de montage partiel, l'élément de base (3) peut être pivoté par rapport à l'élément de pression (7)

6. Dispositif selon l'une des revendications 1 à 5, **caractérisé en ce que** l'élément de pression (7) présente une butée pour les talons.

7. Dispositif selon l'une des revendications 1 à 6, **caractérisé en ce que** l'élément de pression (7) est réalisé, au moins par sections, sous la forme d'un profilé en L.

8. Dispositif selon l'une des revendications 1 à 7, **caractérisé en ce que** l'élément de pression (7) est guidé dans la région de la toise (1) par au moins un élément d'alignement (15).

9. Dispositif selon la revendication 8, **caractérisé en ce que**, dans la région de l'élément d'alignement (15), est agencé au moins un ressort (16).

10. Dispositif selon la revendication 8, **caractérisé en ce que** l'élément de pression (7) est guidé dans la région de la toise (1) par au moins deux éléments d'alignement (15).

11. Dispositif selon l'une des revendications 8 à 10, **caractérisé en ce que** l'élément d'alignement (15) est disposé dans un espace, qui est entouré, au moins par sections, par une paroi (13) de la toise (1).

12. Dispositif selon l'une des revendications 8 à 10, **caractérisé en ce que** l'élément d'alignement (15) est disposé en dehors d'une paroi (13) de la toise (1).

13. Dispositif selon l'une des revendications 1 à 12, **caractérisé en ce que** l'élément de pression (7) est fixé sur l'élément de base (3) et est conçu pour agir sur la toise (1).
